(19) Europäisches Patentamt
European Patent Office
Office européen des brevets

(11) Publication number: **0 048 532**
**A2**

(12) # EUROPEAN PATENT APPLICATION

(21) Application number: 81201298.7

(22) Date of filing: 20.11.81

(51) Int. Cl.³: **C 07 D 327/04**
C 07 D 239/55
//C07C149/243, C07C143/155,
C07D263/22

(43) Date of publication of application:
31.03.82 Bulletin 82/13

(84) Designated Contracting States:
AT BE CH DE FR GB IT LI LU NL SE

(71) Applicant: Stichting Research en Techniek van de Katholieke Universiteit
Toernooiveld 1
NL-6525 ED Nijmegen(NL)

(72) Inventor: Ottenheijm, Henricus Carl Jozeph
Gagelveld 5
NL-6596 CC Milsbeek(NL)

(72) Inventor: Liskamp, Rob Matthias Joseph
Kwartelstraat 31
NL-6601 CE Wychen(NL)

(74) Representative: Urbanus, Henricus Maria, Ir. et al,
c/o Vereenigde Octrooibureaux Nieuwe Parklaan 107
NL-2587 BP 's-Gravenhage(NL)

(54) **Sultines; a process for producing these; a process for producing sulfoxides; a process for producing sparsomycin and related compounds.**

(57) The invention provides novel sultines which are functionalized and satisfy formula 1, in particular formula 2. The invention also provides a particular process for producing such sultines from compounds having formula 4 or 5, and a process for producing compounds having formula 8, in particular formula 9, by stereo-specific ring-opening of such sultines. The invention further provides an efficient chemical route for producing sparsomycin and related compounds.

EP 0 048 532 A2

Croydon Printing Company Ltd.

Title:

Sultines; a process for preparing these; a process for producing sulfoxides; a process for producing sparsomycin and related compounds.

------

This invention relates to sultines and a process for producing these.

The invention also relates to a process for producing sulfoxides and to a process for producing sparsomycin and related compounds.

A limited number of sultines are known. A survey of known sultines is to be found in N.K. Sharma et al., Can. J. Chem. 1976, 54, 3012.

In E.N. Givens et al., J. Org. Chem. 1967, 32, 2857, sultines are described having formula 18, in which the phenyl group may be substituted by one or two chlorine atoms.

Sultines having formula 19 are known from R.M. Dodson et al., J. Chem. Soc., Chem. Commun. 1968, 9.

These publications describe a processs for the production of sultines in which 1-mercapto-propanol-3 compounds are reacted with $Cl_2$ and excess acetic acid.

The present invention provides functionalized sultines having formula 1, wherein X = O or NH; $Y^1$ = H or a protective group; m = 1 or 2; n = 1 or 2; and salts thereof.

In particular, the invention provides sultines having formula 2 wherein $Y^1$ = H, t-butyloxycarbonyl (BOC) or benzyloxycarbonyl (Cbo) and acid addition salts thereof, wherein $Y^1$ = H.

These new compounds contain 2 chiral (i.e. asymmetric) atoms,

namely, a chiral C atom and a chiral S atom. The invention comprises both mixtures of sultine diastereomers and the individual diastereomers.

These new sultines have a number of important and surprising properties. As a result of their chiral character, in which they are sharply distinguished from the corresponding lactones, the sultine ring can be opened in a diastereo-selective manner. As a result of the particular ring structure, the ring oxygen atom (i.e. the alcohol function) is protected by the sulphur atom, and the sulphur atom is rendered accessible by the ring oxygen atom for a nucleophilic attack. These properties make the sultines according to the invention excellently suitable as starting materials for the production of interesting chemical compounds, in particular for the production of certain diastereomers of such compounds. In this connection one important advantage of the sultines according to the invention is that the sultine diastereomers can very easily be separated from each other on account of their exceedingly large difference in polarity, for example by column chromatography. A further advantageous property of the sultines according to the invention is that each diastereomer can readily be epimerized to a mixture of diastereomers, for example, by heating at approximately 130°C, or by other means.

Thus one of the sultine diastereomers, e.g. $S_c$-$S_s$, can be treated with two equivalents of $NaOCH_3$ in $CH_3OH$, to produce a 1 : 1 mixture of the starting diastereomer $S_c$-$S_s$ and the diastereomer $S_c$-$R_s$. One possible explanation of this epimerization is a transesterification reaction of the $S_c$-$R_s$ methylsulfinate ester produced by ring opening to the corresponding $S_c$-$S_s$ methylsulfinate ester and ring closure thereof. Thus the undesired diastereomer isolated by column chromatography

can be converted into a mixture containing the desired diastereomer, from which mixture a further quantity of the desired diastereomer can be recovered by separation. By repeating epimerization and separation the yield of the desired diastereomer can be maximized.

The sultine $S_c$-$R_s$ diastereomer having formula 3, in which $Y^1$ = H, BOC or Cbo, and acid addition salts thereof, in which $Y^1$ = H, is particularly preferred, because it can be used for preparing $S_c$-$R_s$ sparsomycin having formula 13, a well-known medicament having strong anti-tumor and antibiotic properties; moreover, this compound plays a central role in research into the protein biosynthesis inhibition.

The invention further provides a particular process for producing the sultines according to the invention, which process is characterized by reacting a compound having formula 4, wherein

X = O or NH,

$Y^1$ = a protective group,

$Y^2$ = H or a group removable by oxidation,

$Y^3$ = H or a group removable by oxidation,

m = 1 or 2,

n = 1 or 2,

or the dimer having formula 5, wherein the symbols have the above meanings, in the presence of a weakly nucleophilic oxygen donor, with an oxidant comprising an oxidatively active moiety and a weakly nucleophilic counter-ion, and if desired removing the protective group $Y^1$, or replacing it by a different protective group, and if desired separating the resulting mixture of diastereomers by column chromatography.

In the starting compounds having formulae 4 and 5, $Y^2$ is preferably H, but may also represent a group that is removed by the

oxidation reaction, for example, methylthiomethyl and tetrahydropyranyl.

In the starting compounds having formula 4, $Y^3$ is preferably H, but may also represent a group that is removed by the oxidation reaction, e.g. -S-alkyl, tert.alkyl, benzyl, alkyl- and/or alkoxy-substituted benzyl, trityl and allyl.

The preferred oxidant in this process is N-chloro-succinimide (NCS), N-bromo-succinimide (NBS), N-chloro-benztriazole, hypochlorous acid (HOCl) or tert.alkyl hypochlorite, in particular tert.butyl-OCl. Of these, NCS and NBS are most preferred: NCS because it is relatively stable, easy to handle and inexpensive, NBS because a larger quantity than the stoichiometric proportion can be used without adverse effects.

According to the invention, the preferred oxygen donor is a carboxylic acid, carboxylic acid anhydride, alcohol, or water. Preferably, acetic acid or acetic acid anhydride is used, preferably in excess, so that it can also serve as a solvent. It is also possible, however, to use an inert solvent, such as an aromatic hydrocarbon, e.g. benzene, or a halogenated hydrocarbon, e.g. $CH_2Cl_2$, $CHCl_3$, $CCl_4$, $CH_2Br_2$, etc. as the only solvent or as a solvent component.

The reaction conditions, that is to say temperature and pressure, are not particularly critical. Preferably, however, the reaction is carried out at room temperature without cooling and at atmospheric pressure. Preferably the mixture is thoroughly stirred during the reaction.

As, generally speaking, it will be desirable to produce a specific sultine diastereomer, the preferred starting product is a specific stereoisomer of the compound having formula 4 or 5, that is to say, the $R_c$ or the $S_c$ stereoisomer. As stated before, the resulting mixture of

$R_s$ and $S_s$ sultines can easily be separated.

In a particularly preferred embodiment of the invention, the starting product is a compound having formula 6, in which $Y^1$ = BOC or Cbo, or the dimer thereof. These compounds can be prepared in a manner known per se from D- and/or L-cystin or -cystein by esterification with thionyl chloride and alcohol, e.g. methanol (gives the methyl ester), introducing the N-protective group, e.g. by means of N-carbobenzoxy-O-N-phthalimide or di-tert.butyl-pyrocarbonate, reduction with $LiBH_4$ and oxidation with $I_2$.

When the protective group $Y^1$ in the compound having formula 6 of the dimer thereof represents the tert.butyloxycarbonyl group, which under certain conditions is preferred, the reaction is preferably carried out in the presence of a non-nucleophilic base, such as pyridine or a trialkylamine, e.g. triethylamine. This prevents the removal of the acid-labile BOC group.This applies generally when using compounds having formula 4 or 5, if $Y^1$ is an acid-labile group.

The reaction is preferably carried out using stoichiometric quantities of the reaction components, except for the oxygen donor, which is preferably used in excess for it to serve as a solvent. Stoichiometric quantities means that 3 equivalents of oxygen (preferably NCS or NBS) are used per 2 equivalents of starting compound having formula 4 or 5 or per equivalent dimer thereof. If the oxidant is NCS, an excess thereof leads to the formation of compounds having formula 14, presumably owing to cleavage of the C-O bond in the sultine ring. On the other hand, when NBS is used in excess, such compounds are not formed in detectable quantities.

As will be elucidated hereinafter, under certain conditions the use of the $S_c$ stereoisomer having formula 7 or the dimer thereof is preferred. This results in a mixture of the sultine diastereomers $S_c$-$S_s$ and $S_c$-$R_s$, from which, by virtue of the large difference in Rf value, the sultine diastereomer $S_c$-$R_s$ having formula 3 can be recovered in a simple manner by column chromatography. By epimerizing the isolated $S_c$-$S_s$ diastereomer and separating the resulting mixture by column chromatography, and if desired repeating this, the yield of $S_c$-$R_s$ diastereomer can be optimized. This diastereomer is a suitable starting compound for the production of the medicament $S_c$-$R_s$ sparsomycin.

The above prior process for producing sultines, using 3 equivalents $Cl_2$ and excess acetic acid produces from the starting compounds having formula 4 or 6, or the dimers thereof, the undesirable product having formula 14. When $SO_2Cl_2$ is used, instead of $Cl_2$, an undesirable result is obtained, too. According to the invention, therefore, the selection of a suitable oxidant and, possibly, the proportion in which it is used, is essential for producing the desired sultine in a good yield.

According to another aspect of the invention, it has been found that the sultines according to the invention can be converted by stereospecific opening of the O-S bond into sulfoxides, which are useful starting compounds for the preparation of interesting chemical compounds, in particular for the preparation of certain diastereomers of such compounds. This nucleophilic ring-opening reaction proceeds with inversion, that is to say, reversal of the chirality of the sulphur atom.

The invention accordingly provides a process for producing compounds having formula 8, wherein X = O or NH, $Y^1$ = H or a protective group, $R^1$ = H, alkyl, acyl, or a protective group, $R^2$ = is a nucleophilic group, m = 1 or 2, and n = 1 or 2, said process being characterized by

reacting a sultine having formula 1, wherein $Y^1$ represent a protective group, and the other symbols have the meanings specified in claim 1, at low temperatures and in an inert atmosphere, with an organolithium compound $LiR^2$ in a solvent conventional for carb-anion reactions at low temperatures, and if desired providing the alcohol function with a protective group or etherifying or esterifying it, and if desired removing the protective group $Y^1$ or replacing it by a different protective group.

Preferably, $R^2$ is selected from the group consisting of alkyl, alkoxyalkyl, alkoxyaryl, alkyl thioalkyl, alkyl thioaryl, alkyl thio-oxoalkyl, alkyl thio-oxoaryl, alkyl thio-dioxoalkyl, and alkyl thio-dioxoaryl. In these compounds, the alkyl groups are preferably lower alkyl groups containg 1-8 carbon atoms, more preferably 1-4 carbon atoms, and most preferably 1-2 carbon atoms.

For that matter, a process for the nucleophilic opening of the sultine ring is known per se. That process, however, concerns non-functionalized sultines, and does not employ organo-lithium compounds. Thus, according to W.H. Pirkle et al., J. Am. Chem. Soc 1976, 98, 1832, a Grignard reagent is used, while according to D.N. Harpp et al., J.Org. Chem. 1976, 41, 3987, organo-copper-lithium compounds are used.

These known reagents turn out to be unsuitable for realizing an efficient nucleophilic and stereospecific ring opening of the functionalized sultines according to the present invention.

Furthermore, the use of organo-lithium compounds, in particular alkyl thiomethyl-lithium, is known from L. Colombo et al., Tet. Letters 1978, 3861. That publication, however, is concerned with the stereospecific production of optically active dithioacetal monosulfoxides from noncyclic optically active sulfinate esters.

The process according to the invention is carried out under conditions conventional for carb-anion reactions, that is to say, beginning at low temperatures in the order of -90 to -60°C, and at atmospheric pressure (not critical) in a conventional solvent, e.g. alkanes, such as hexane, ethers, such as tetrahydrofuran, in an inert atmosphere, for example argon or nitrogen, to prevent the presence of water, oxygen and carbon dioxide. Preferably the mixture is stirred well during the reaction. To produce a high yield and purity, it is desirable for the reaction mixtures to be acidified rapidly after completion of the reaction.

According to one embodiment of this process according to the invention, a mixture of sultine diastereomers is used, and the resulting mixture of $S_s$-$R_s$ diastereomers of the compound having formula 8 is separated by column chromatography. Under certain conditions, this method may be preferred. This may in particular be the case if the diastereomers of the compound having formula 8 in question exhibit a large difference in Rf value, and if successful epimerization of the undesirable diastereomer is possible, for example,by using acid conditions.

Mostly, however, the use of a specific sultine diastereomer will be preferred, because the sultine diastereomer can at any rate quite easily be separated, and successful epimerization of the undesirable diastereomer is possible, while the reaction with $R^2Li$ is stereospecific, that is to say, no trace of the corresponding diastereomers is found.

In a preferred embodiment of the invention, a compound having formula 9 is prepared by starting from a sultine having formula 2 in which $Y^1$ = BOC or Cbo. In particular if $Y^1$ = Cbo, the alcohol function of the compound having formula 9 is preferably provided with a protective group, e.g. the tetrahydropyranyl group.

Preferably, the $S_c$-$R_s$ diastereomer having formula 10 is prepared, as this known per se compound is a suitable starting material for the production of the medicament $S_c$-$R_s$ sparsomycin.

According to another aspect of the invention, it has been found that the new functionalized sultines according to the invention make possible a novel and efficient chemical route for producing sparsomycin and related compounds.

Sparsomycin ($S_c$-$R_s$) is a metabolite of Streptomyces sparsogenes and Streptomyces cuspidosporus. It is active against several tumors, bacteria, fungi and viruses; and is of great importance to research into the inhibition of protein biosynthesis.

Natural sources only give a limited accessibility, and fail to give strudurally related compounds that may be of great significance in connection with their properties and/or for research purposes. For these reasons, there is a great need for a flexible chemical overall synthesis. Two chemical overall syntheses have meanwhile been disclosed.

Thus P. Helquist et al., J. Am. Chem. Soc. 1979, 101, 1057, describe a process for preparing sparsomycin, which comprises preparing, starting from D-cystein, an α-sulfinyl carbanion having formula 15, which by sulphenylation with $(CH_3S)_2$ is converted into a mono-oxo-dithio-acetal having formula 9, in which $Y^1$ and $R^1$ are protective groups and $R^2$ represents methyl thiomethyl, and from which, after the removal of the protective groups, sparsomycin is produced by reaction with the compound having formula 12.

Another process for producing sparsomycin is described by H.C.J. Ottenheijm, R.M.J. Liskamp et al., J. Org. Chem. 1981, 46, 3273. In it, starting from D-cystein, a cysteinol-α-halosulfoxide having for-

mula 16 is produced, which is reacted with sodium methyl mercaptide ($CH_3SNa$) to form the mono-oxo-dithio-acetal having formula 9, in which $Y^1$ and $R^1$ are protective groups and $R^2$ represents methyl thiomethyl, which compound, after the removal of the protective groups, is reacted with the compound having formula 12.

The present invention provides a process for producing sparsomycin and related compounds having formula 12, in which $R^1$ = H, alkyl, acyl; and $R^2$ is selected from alkyl, alkoxyalkyl, alkoxyaryl, alkyl thioalkyl, alkyl thioaryl, alkylthio-oxoalkyl, alkylthio-oxoaryl, alkylthio-dioxoalkyl and alkylthio-dioxoaryl, and which comprises reacting a compound having formula 12, or a suitable derivative thereof, with a compound having formula 9, wherein $Y^1$ = H and $R^1$ and $R^2$ have the above meanings, said compound being produced by the process according to claim 22 or 23.

Preferably, according to the invention, a sultine having formula 2 is prepared by the process according to one or more of claims 5-17, from this a compound having formula 9 is prepared by the process according to one or more of claims 18-23, the protective group $Y^1$ is removed, and thereafter a reaction with the compound having formula 12 or a suitable derivative thereof is carried out.

Preferably the procedure is such that one of the trans-diastereomers $S_c$-$R_s$, $S_c$-$S_s$, $R_c$-$R_s$, $R_c$-$S_s$ of the compound having formula 11 is prepared by starting from the corresponding diastereomer of the compound having formula 9, and that the resulting trans-diastereomer is, if desired, converted into the cis-diastereomer by irradiation.

Preferably, trans-$S_c$-$R_s$-sparsomycin having formula 13 is prepared.

The last step of the total synthesis according to the invention, namely, the reaction of the amino alcohol mono-oxo-dithioacetal having formula 10 (or stereomers thereof) with the compound having formula 12, is known per se, for example, from the above publication by H.C.J. Ottenheijm, R.M.J. Liskamp et al. The contents of that publication, in which, among other things, the removal of the protective group $Y^1$ and the preparation of the compound having formula 12 are disclosed, is inserted herein by reference.

The route according to the invention to sparsomycin and related compounds has a number of decisive advantages as compared with the known routes described above. First of all, one can dispense with the introduction and subsequent removal of a protective group for the hydroxyl function ($Y^2$ in formulae 15 and 16). As a result, the required number of steps is smaller. Moreover, each new step by itself is better than the comparable steps in the known routes. Furthermore, a very important advantage resides in the possibility of effecting a good separation of the diastereomers, and optimizing the yield by epimerization on the S atom of the undesirable diastereomer.

The invention will be explained in more detail by means of the examples. Beforehand, the following can be stated about the methods of analysis and separation used.

'H-NMR spectra were measured on Varian Associates Model T-60 or a Bruker WH-90 spectrometer using TMS or tert.butanol as the internal standard. Unless stated otherwise, $CDCl_3$ was used as the solvent.

$^{13}C$-NMR spectra were measured on a Bruker WP-60 spectrometer.

IR spectra were measured using a Perkin Elber spectrophotometer, model 997 and UV spectra on a Perkin Elber spectrophotometer, model 555.

The specific rotation was determined using a Perkin Elmer polarimeter model 241.

Circular dichroism spectra were measured using a Dichrograph II apparatus (Roussel-Jouan, France).

Mass spectra were obtained with a double-focusing Varian Associates SMI-B spectrometer.

Melting points were determined on a Köfler melting point apparatus (Leitz-Wetzlar) and were not corrected.

Thin-layer chromatography (TLC) was carried out using Merck silicagel F-254 plates having a thickness of 0.25 mm. The following solvent systems were used:

A: methanol/$CH_2Cl_2$, 1/9 (v/v)

B: methanol/$CH_2Cl_2$, 6/94 (v/v)

C: methanol/$CH_2Cl_2$, 4/96 (v/v)

D: methanol/$CH_2Cl_2$, 3/97 (v/v)

Spots were visualized with a UV lamp, iodine vapour, ninhydride or $Cl_2$-TDM.

For column chromatography, Merck silicagel H (type 60) was used.

The miniprep LC (Jobin Yvon) was used for preparative HPLC.

Example I

a) Preparation of N-benzyloxycarbonyl-D-cystinol

To a stirred, cooled (-78°C) solution of sodium borohydride (6.81 g, 180 mmol) and lithium iodide (24.09 g, 180 mmol) in 600 ml of dry dimethoxyethane (DME), N-benzyloxycarbonyl-D-cystin-methyl ester (16.1 g, 30 mmol) was added in one portion, the ester being prepared in the manner described by E.L.Gustus, J.Org.Chem 1967, 32, 3425.

The reaction mixture was allowed to rise in temperature to room temperature, and was then stirred until the reaction was complete, as monitored by TLC (solvent system A). The solution was neutralized to pH 7 with an aqueous solution of 1N HCl with ice-cooling. Stirring was continued for 1 hour at room temperature, after which time the volume was reduced to one-half. A methanolic solution of 0.1 M iodine and 0.2 M pyridine was added until a faint-yellow colour of iodine persisted.

The excess of iodine was destroyed by adding a few crystals of $Na_2S_2O_5$. After the evaporation of DME and methanol in vacuo, water and dichloromethane were added. The aqueous layer was extracted three times with dichloromethane and twice with ethyl acetate.

The combined organic layers were dried over $Na_2SO_4$, and the solvent was evaporated in vacuo. After recrystallization of the residue from ethyl acetate, 12.53 g (yield 87%) of the N-benzyloxycarbonyl-D-cystinol was obtained. This material was homogenous on TLC (Rf 0.17, solvent system B).

NMR($CD_3OD$) $\delta$2.67-3.13 (m,2H,$CH_2S$), 2.63(br.d,2H,$CH_2O$), 3.80-4.10 (m,1H,$C\underline{H}CH_2$), 5.07(s,2H,$C_6H_5C\underline{H}_2$), 7.32(s,5H,$C_6H_5$); IR(kBr) 3300,1695, 1680, 1535 $cm^{-1}$; Anal. calculated for $C_{22}H_{28}N_2O_6S_2$: C: 54.98; H: 5.87; N: 5.83. Found: C: 55.27; H: 5.84; N: 5.65.

b) Preparation of 4(N-benzyloxycarbonyl)amino 1,2-oxathiolane-2-oxide.

To a stirred solution of N-benzyloxycarbonyl-D-cystinol (4.80 g, 10 mmol) in 100 ml glacial acetic acid, a solution of N-chlorosuccinimide (4.01 g, 30 mmol) in 150 ml glacial acetic acid was added dropwise at room temperature. The reaction mixture was stirred overnight. After completion of the reaction, as monitored by TLC (solvent system B), the acetic acid was evaporated in vacuo at room temperature. The residue was

dissolved in 400 ml di-chloromethane and 15 ml water. The organic layer was separated and dried, and the solvent evaporated in vacuo. The residue was dried and then chromatographed over silica /eluent methanol/$CH_2Cl_2$, 0.5/99.5 (v/v)/, to yield $S_c$-$R_s$ sultine (45%) and $S_c$-$S_s$ sultine (45%).

The synthesis of these sultines with N-bromosuccinimide (3 equivalents or more) was carried out as described above. After evaporation of acetic acid, residual bromine was removed by dissolving the residue in methanol and evaporating the solvent in vacuo; this treatment was repeated twice. Column chromatography of the residue gave the $S_c$-$R_s$-sultine (43% yield) and the $S_c$-$S_s$ sultine (43% yield).

$S_c$-$R_s$ Cbo sultine: melting point 87°C (AcOEt hexane) $R_f$ 0.43 (solvent system B): NMR δ3.24 and 3.07 /AB part of ABX spectrum, 8 lines, $J_{AB}$ = 13.9 Hz, $J_{AX}$ = 6.2 Hz, $J_{BX}$ = 2.4 Hz, 2H, $CH_2S(O)$/, 4.38-4.52 and 4.62-4.88 (m,3H,$CHCH_2O$), 5.09(s,2H,$C_6H_5CH_2$), 5.45(br.d,1H,NH), 7.33(s,5H, $C_6H_5$); IR(kBr)3310, 1720, 1530, 1110 $cm^{-1}$; exact mass calculated for $C_{11}H_{13}NO_4S$: 255.147. Found: 255.149; Analysis calculated for $C_{11}H_{13}NO_4S$: C: 51.75; H: 5.13; N: 5.49. Found: C: 51.89; H: 5.11; N: 5.35.

$S_c$-$S_s$ Cbo sultine: $R_f$ 0.64 (solvent system B): NMR δ2.91 and 3.20 /AB part of ABX spectrum, 8 lines, $J_{AB}$ = 13.9 Hz, $J_{AX}$ = 6.2 Hz, $J_{BX}$ = 2.4 Hz, 2H, $CH_2S(O)$/, 4.58 and 4.75 (AB part of ABX spectrum, 8 lines, $J_{AB}$ = 9.6 Hz, $J_{AX}$ = 5.7 Hz, $J_{BX}$ = 1.9 Hz, 2H, $CH_2O$), 4.98 (m,1H,$CHCH_2O$), 5.10(s,2H,$C_6H_5CH_2$), 6.36(br.d,1H,NH), 7.33(s,5H,$C_6H_5$); IR(kBr)3320, 1690, 1545, 1108 $cm^{-1}$; exact mass calculated for $C_{11}H_{13}NO_4S$: 255.147. Found: 255.147; Anal. calculated for $C_{11}H_{13}NO_4S$: C: 51.75; H: 5.13; N: 5.49. Found: C: 51.42; H: 5.20; N: 5.35.

Comparative example 1

Preparation of 3-chloro-2-(N-benzyloxycarbonyl)amino-1-propane sulfonyl chloride.

To a stirred solution of N-benzyloxycarbonyl-D-cystinol (1.44 g, 3 mmol) in 40 ml glacial acetic acid, a solution of chlorine (1.59 g, 22 mmol) in 15 ml dry, ethanol-free dichloromethane was added in small portions at room temperature. After completion of the addition, the reaction mixture was stirred for two hours at room temperature, after which time the excess of chlorine was removed by a stream of argon. Evaporation of the solvent at room temperature in vacuo gave the title compound in a yield of 78%.

$R_f$ 0.25 (solvent system B); NMR δ3.67-4.00 (m,2H,$CH_2SO_2Cl$), 4.00-4.10 (br.d,2H,$CH_2Cl$), 4.44-4.82(m,1H,$\underline{CH}CH_2Cl$), 5.13(s,2H,$C_6H_5\underline{CH_2}$), 5.59 (br.d, 1H,NH), 7.35(s,5H,$C_6H_5$); IR(nujol)3340, 1695, 1380, 1360, 1350, 1175 $cm^{-1}$, mass spectrum, m/e 325, 327, 329($M^+$).

Example II

a) Preparation of N-tert-butyloxycarbonyl-D-cystinol

N-tert.butyloxycarbonyl-D-cystine methylester (7.03 g, 14 mmol), prepared in the manner described by H.C.J. Ottenheijm, R.M.J. Liskamp, et al., in J.Org.Chem. 1981, 46, 3273, was reduced with lithium borohydride (3.41 g, 90 mmol sodium borohydride and 12.05 g, 90 mmol lithium iodide in 200 ml dry DME) in the manner described in Example Ia. Processing was modified, however, due to the acid lability of the N-protecting group. pH was adjusted to 5 by addition of aqueous 1N $KHSO_4$ to the stirred and cooled (0°C) solution. Sometimes a sticky mass precipitated before neutralization was complete. In that case the solvent was evaporated in vacuo, the residue was dissolved in methanol/water (1/1, v/v), and the

neutralization was then completed. The oxidation with iodine was carried out as described in Example Ia. Thereafter the methanol was evaporated *in vacuo*, and water and ethyl acetate were added. The aqueous phase was extracted five times with ethyl acetate. The combined organic layers were washed with brine and dried over $Na_2SO_4$, and the solvent was evaporated *in vacuo*. The residue was re-crystalized from methanol/water, to produce N-tert.butyloxycarbonyl-D-cystinol in a yield of 87%; melting point 124-125°C; $R_f$ 0.23 (solvent system A); NMR($CD_3OD$) δ1.41 (s,9H,t-Bu), 2.85 (d,2H,$CH_2S$), 3.46-4.05(m,3H,$\underline{CHCH_2}O$); IR (kBr): 3360, 3600-3100, 1685, 1525 $cm^{-1}$; Anal. calculated for $C_{16}H_{32}N_2O_6S_2$: C: 46.58; H: 7.82; N: 6.79. Found: C: 46.78; H: 7.92; N: 6.87.

b) Preparation of 4(N-tert.butyloxycarbonyl)amino-1,2-oxathiolane-2-oxide

A solution of N-chlorosuccinimide &4.01 g, 30 mmol) in 150 ml of glacial acetic acid was added dropwise to a solution of N-tert.butyloxycarbonyl-D-cystinol (4.12 g, 10 mmol) and pyridine (2.4 g, 30 mmol) in 100 ml glacial acetic acid. Following the procedure as described in Example Ib, N-tert.butyloxycarbonyl-sultine diastereomers $S_c$-$R_s$ and $S_c$-$S_s$ were isolated in a 1/1 ratio (yield 86%).

$S_c$-$R_s$ BOC sultine: melting point 127°C ($CH_2Cl_2$-$CCl_4$); $R_f$0.43 (solvent system B); 'H-NMR δ1.44 (s,9H,t-Bu), 3.14 and 3.45 (AB part of ABX spectrum, $J_{AX}$ = 2.9 Hz, $J_{BX}$ = 6.0 Hz, $J_{AB}$ = 13.9 Hz, 2H, $CH_2S$), 4.33-4.98 (m.2H,$\underline{CH_2}O$), 4.79 [m,1H (covered by $\underline{CH_2}O$) $\underline{CH}CH_2O$], 5.05(br,1H,NH); IR (kBr): 3325, 1683, 1530, 1102 $cm^{-1}$; $^{13}C$-NMR($CD_2Cl_2$): δ28.4($\underline{C}H_3)_3C$); $\underline{C}$-N covered by $CD_2Cl_2$ signals. 67.4($\underline{C}S$), 80.7 $(CH_3)_3\underline{C}$), 77.6 [$\underline{C}OS(O)$], 155.2 [$\underline{C}(O)$]; exact mass calculated for $C_7H_{12}NO_4S$ ($M^+$-$CH_3$): 206.139, found 206.139; Anal. calculated for $C_8H_{15}NO_4S$: C: 43.42; H: 6.83; N: 6.33. Found: C: 43.46; H: 6.90; N: 6.13.

$S_c$-$S_s$ BOC sultine: melting point 135-136°C ($CH_2Cl_2$) $R_f$ 0.64 (solvent system B); 'H-NMR δ1.43 (s,9H,t-Bu), 2.94 and 3.22 (AB part of ABX spectrum, $J_{AX}$ = 1.3 Hz, $J_{BX}$ = 6.6 Hz, $J_{AB}$ = 9.7 Hz, 2H, $CH_2S$), 4.59 and 4.76 (AB part of ABX spectrum, $J_{AX}$ = 1.9 Hz, $J_{BX}$ = 5.5 Hz, $J_{AB}$ = 9.7 Hz, 2H, $CH_2O$), 4.85(m,1H,$\underline{C}HCH_2O$), 6.06(br,1H,NH); $^{13}$C-NMR($CD_2Cl_2$): δ28.5/$(\underline{C}H_3)_3C$/, 50.9(C-N), 62.8(CS), 80.2$(CH_3)_3\underline{C}$), 83.0/$\underline{C}$OS(O)/, 155.2/$\underline{C}$(O)N/; IR(kBr) 13325,1725,1525,1115 $cm^{-1}$; exact mass calculated for $C_7H_{12}NO_4S$ ($M^+$-$CH_3$): found 206.139; Anal. calculated for $C_8H_{15}NO_4S$: C: 43.42; H: 6.83; N: 6.33. Found C: 43.56; H: 6.85; N: 6.29.

Example III

Preparation of N-benzyloxycarbonyl-S-oxo-S(methylthiomethyl)-D-cysteinol and N-tert.butyloxycarbonyl-S-oxo-S(methylthiomethyl)-D-cysteinol

In order to prepare the anion of dimethyl sulfide, tetramethyl ethylene diamine (0.70 g, 0.91 ml, 6.0 mmol), freshly distilled dimethyl sulfide (0.37 g, 0.44 ml, 6.0 mmol) and 2 ml freshly distilled THF were respectively introduced by means of a syringe into a 50 ml, cooled (0°C) round-bottom flask (equiped with a septum), containing 3.75 ml of a 1.6M solution of n-butyllithium in hexane (6 mmol). The resulting solution was stirred at room temperature for four hours, cooled to -30°C, and added dropwise to a cooled (-78°C) solution of the N-benzyloxycarbonyl-sultine diastereomers $S_c$-$R_s$ and $S_c$-$S_s$ (510 mg, 2.0 mmol) in 6 ml freshly distilled THF.

Subsequently the reaction mixture was stirred at -70°C for 30 minutes, stirred at room temperature for 30 minutes, and thereafter rapidly quenched at 0°C with 5 ml of a saturated aqueous solution of $KHSO_4$. Immediately thereafter the pH of the mixture was adjusted to 6-8 by adding solid sodium carbonate. Ethyl acetate was added and thereafter

the water layer was extracted four times with ethyl acetate. The combined organic layers were dried over $Na_2SO_4$, and the solvent was evaporated *in vacuo*. The residue was chromatographed at a slightly elevated pressure (10 mm Hg, eluent methanol/$CH_2Cl_2$, 5/95, v/v), to produce $S_c$-$R_s$ N-benzyloxycarbonyl-S-oxo-S(methylthiomethyl)-D-cysteinol (yield 46%) and $S_c$-$S_s$ N-benzyloxycarbonyl-S-oxo-S(methylthiomethyl)-D-cysteinol (yield 33%).

Prepared in the same way were the compounds $S_c$-$R_s$ N-tert. butyloxycarbonyl-S-oxo-S(methylthiomethyl)-D-cysteinol, in a yield of 71%, and $S_c$-$S_s$ N-tert.butyloxycarbonyl-S-oxo-S(methylthiomethyl)-D-cysteinol in a yield of 70%, after preparative HPLC (solvent system C). These compounds were in every respect identical to the corresponding compounds described earlier by H.C.J. Ottenheijm, R.M.J. Liskamp et al., J.Org. Chem. 1981, 46, 3273.

$S_c$-$R_s$ N-benzyloxycarbonyl-S-oxo-S(methylthiomethyl)-D-cysteinol: Melting point 93°C ($CH_2Cl_2$-hexane); $R_f$ 0.35 (solvent system A); NMR($CD_2Cl_2$) δ2.27 s,3H,$SCH_3$), 2.96 and 3.33 (AB part of ABX spectrum, $J_{AX}$ = 4.9 Hz, $J_{BX}$ = 5.8 Hz, $J_{AB}$ = 13.4 Hz), 3.60-3.95(m,2H,$CH_2OH$), 3.71 and 3.84 (AB spectrum covered by $CH_2OH$, 2H, $J_{AB}$ = 13.5 Hz, S(O)$CH_2$S), 4.26(m,1H,$CHCH_2$O), 5.09 (s,2H,$C_6H_5CH_2$), 5.94(br.d,1H,NH), 7.34(s,5H,$C_6H_5$); IR(kBr)3335, 1680, 1530, 1006 $cm^{-1}$; exact mass calculated for $C_{13}H_{19}NO_4S_2$: 317.256. Found: 317.256; Anal. calculated for $C_{13}H_{19}NO_4S_2$: C: 49.19; H: 6.03; N: 4.41. Found: C: 49.15; H: 6.03; N: 4.34.

$S_c$-$S_s$ N-benzyloxycarbonyl-S-oxo-S(methylthiomethyl)-D-cysteinol: Melting point 162°C ($CH_2Cl_2$-hexane); $R_f$ 0.32 (solvent system A); NMR ($CD_2Cl_2$) δ2.29 (s,3H,$SCH_3$), 2.97 and 3.21 (AB part of ABX spectrum, $J_{AX}$ = 7.0 Hz, $J_{BX}$ = 6.4 Hz, $J_{AB}$ = 13.2 Hz, 2H, $CHCH_2$S(O)), 3.73-4.00(m,4H,$CHCH_2$O and S(O)$CH_2$S), 4.16(m,1H,$CHCH_2$O), 5.09(s,2H,$C_6H_5CH_2$), 5.70(br,1H,NH), 7.35

(s,5H,$C_6H_5$); IR(kBr)3330, 1695, 1538, 1025, 1015 $cm^{-1}$; exact mass calculated for $C_{13}H_{19}NO_4S_2$: 317.256. Found: 317.257; Anal. calculated for $C_{13}H_{19}NO_4S_2$: C: 49.19; H: 6.03; N: 4.41. Found: C: 49.16; H: 5.95; N: 4.16.

Comparative example 2

Preparation of 2-oxo-4-(methylthiomethyl sulfoxomethylene)oxazolidine (formula 17)

The ring-opening reaction of $S_c$-$R_s$ Cbo sultine or the $S_c$-$S_s$ Cbo sultine was carried out as described in Example III, but with slightly different processing. Instead of quenching with a saturated aqueous solution of $KHSO_4$, the reaction mixture was stirred overnight with solid $KHSO_4$. Methanol was then added, and the mixture was stirred for another two hours at room temperature. After removal of the salts by filtration, the filtrate was concentrated to dryness, and the residue was chromatographed over silica (eluent methanol/$CH_2Cl_2$, 5/95, v/v) to produce $S_c$-$R_s$ 2-oxo-4(methylthiomethyl sulfoxomethylene)oxazolidine in a yield of 19% and $S_c$-$S_s$ 2.oxo-4(methylthiomethyl sulfoxomethylene) oxazolidine in a yield of 11%, respectively.

$S_c$-$R_s$ 2-oxo-4(methylthiomethyl sulfoxomethylene)oxazolidine: $R_f$ 0.24 (solvent system A); NMR($CD_2Cl_2$) δ2.31(s,3H,$SCH_3$), 2.89-3.29 (br.d,2H, $CHCH_2S$), 3.77 and 3.90 (AB spectrum, $J_{AB}$ = 13.8 Hz, 2H, S(O) $CH_2S$), 4.10 and 4.69(m,3H,$CHCH_2O$), 6.40(br.s,1H,NH); IR(nujol)3250, 1745, 1710, 1040 $cm^{-1}$; mass spectrum m/e 209 ($M^+$).

$S_c$-$S_s$ 2-oxo-4(methylthiomethyl sulfoxomethylene)oxazolidine: $R_f$ 0.22 (solvent system A); NMR($CD_2Cl_2$) δ2.32(s,3H,$SCH_3$), 2.94-3.29 (m,2H,$CHCH_2S$), 3.78 and 3.86 (AB spectrum, $J_{AB}$ = 13.6 Hz, 2H, S(O)$CH_2$ S), 4.17-4.69(m,3H, $CHCH_2O$), 6.84(br.s,1H,NH); IR(nujol)3240, 1760, 1710, 1045 $cm^{-1}$; mass spectrum m/e 209 ($M^+$).

Example IV

Preparation of N-benzyloxycarbonyl-S-oxo-S-n-butyl-D-cysteinol and N-tert.butyloxycarbonyl-S-oxo-S-n-butyl-D-cysteinol

A cooled ($CO_2$/isopropanol) solution of the n-butyl-lithium-tetramethylethylene diamine complex, prepared by adding tetramethylethylene diamine (523 mg, 0.68 ml, 4.5 mmol) to a solution of n-butyl lithium in hexane (4.5 mmol), was added to a stirred, cooled (-78°C) solution of the $S_c$-$R_s$ Cbo-sultine (383 mg, 1.5 mmol) or $S_c$-$S_s$ BOC sultine (331 mg, 1.5 mmol) in 5 ml freshly distilled, dry THF. The reaction mixture was stirred at -70°C for 30 minutes, and at room temperature for another 30 minutes. Processing was carried out in the manner described in Example III. The title compounds were obtained after HPLC (solvent system C) in yields of 55% and 37%, respectively.

$S_c$-$S_s$ N-benzyloxycarbonyl-S-oxo-S-n-butyl-D-cysteinol: $R_f$ 0.33 (solvent system A); NMR δ0.95(t,3H,$CH_2\underline{CH_3}$), 1.10-2.0(m,4H,S(O)$CH_2\underline{CH_2}\underline{CH_2}CH_3$), 2.52-3.29(m,4H,$CH_2$S(O)$CH_2$), 3.57-3.97(m,2H,$CH\underline{CH_2}$O), 3.97-4.40(m,1H, $\underline{CH}CH_2$O), 5.09(s,2H,$C_6H_5\underline{CH_2}$), 5.88(br,1H,NH), 7.34(s,5H,$C_6H_5$); IR(kBr) 3430, 3200, 1715, 1510, 1060 $cm^{-1}$; exact mass calculated for $C_{15}H_{23}NO_4S$: 313.225. Found: 313.226.

$S_c$-$R_s$ N-tert.butyloxycarbonyl-S-oxo-S-n-butyl-D-cysteinol: $R_f$ 0.32 (MeOH/$CH_2Cl_2$, 9/91, v/v); NMR($CD_2Cl_2$) δ0.97(t,3H,$CH_2\underline{CH_3}$), 1.42 (s,9H,t-Bu), 1.22-1.93(m,4H,S(O)$CH_2\underline{CH_2}\underline{CH_2}CH_3$), 2.62-3.21(m,4H,$CH_2$S (O)$CH_2$), 3.78 (t,2H,$\underline{CH_2}$OH), 3.87-4.27(m,1H,$\underline{CH}CH_2$O), 5.44(br,1H,NH); IR (kBr) 3430, 1710, 1530, 1060 $cm^{-1}$; mass spectrum: m/e 222($M^+$-t-Bu); Anal. calculated for $C_{12}H_{25}NO_4S$: C: 51.59; H: 9.02; N: 5.01. Found: C: 51.53; H: 8.97; N: 4.99.

Example V

Preparation of $S_c$-$R_s$ sparsomycin

a) A solution of the BOC $S_c$-$R_s$ diastereomer produced in Example III (1.273 g, 4.5 mmol) in 50 ml TFA was stirred at $0^oC$ for 30 minutes, whereafter the TFA was evaporated *in vacuo*. The residue was dried *in vacuo* over KOH for 1 hour, and thereafter dissolved in a minimum quantity of water. The solution was applied to an ion exchange column (Amberlite IRA-410, 20-50 mesh, $^-OH$ form).
Elutriation with water and removal of the solvent by freeze drying gave the compound having formula 10 in quantitative yield (823 mg). The resulting product was homogeneous on thin-layer chromatography.
($R_f$ 0.31; sec.butanol/$NH_4OH$ 5/2, v/v); NMR ($CDCL_3/CD_2Cl_2$) $\delta$2.34 (s,3H, $SCH_3$), 2.88-3.00 /AB part of ABX spectrum, 2H, $CHCH_2S(O)$/, 3.33-3.71 (m,3H,$CHCH_2O$), 3.73 and 3.81 /AB spectrum, 2H, J = 13.8 Hz, $S(O)CH_2SCH_3$).
Anal. calculated for $C_5H_{13}NO_2S_2$: C: 32.76; H: 7.15; N: 7.64. Found: C: 32.52; H: 7.19; N: 7.63.

b) Ethyl chloroformate (212 mg, 1.95 mmol) was added to a stirred, cooled ($0^oC$) solution of the acid having formula 12 (383 mg, 1.95 mmol) and triethylamine (218 mg, 2.15 mmol) in 25 ml THF/DMF (1/1, v/v). Stirring was continued at $0^oC$ for 4 hours. Thereafter a solution of the amino alcohol produced under a) (275 mg, 1.5 mmol) in 25 ml THF/DMF (1/1, 4/4) was added dropwise. The reaction mixture was stirred at room temperature for 48 hours, and thereafter the solvents were removed *in vacuo* at room temperature. The resulting product was purified by gel filtration with Sephadex LH-20 (eluent $H_2O$/methanol, 15'85, v/v) to produce $S_c$-$R_s$ sparsomycin in a yield of 48%. It was homogeneous on thin-layer chromatograph ($R_f$ 0.28; methanol/$CH_2Cl_2$, 1/4, v/v); NMR ($D_2O$)

δ2.71 (s,3H, $SCH_3$), 2.82 [s,3H, C(6)$CH_3$), 3.62 (d,2H,$CHCH_2S(O)$], 4.18 (m,2H,$CHCH_2OH$), 4.36 and 4.52 (AB spectrum, J = 13.8 Hz, 2H, $S(O)CH_2SCH_3$), 4.88(m,1H,$CHCH_2OH$), 7.47 and 7.81 (AB spectrum, J = 15.6 Hz,2H,HC = CH); IR (KBr) 1715, 1660, 1600, 1015 $cm^{-1}$; UV(MeCN) $\lambda_{max}$ 297 nm; $[\alpha]_D^{25}$ + 75° (c 0.245, water). Anal. calculated for $C_{13}H_{19}N_3O_5S_2$: C: 43.20; H: 5.30; N: 11.63. Found: C: 43.61; H: 5.20; N: 11.43.

CLAIMS

1. Sultines having formula 1 of the sheet of formulae, wherein

$X_1$ = O or NH,

$Y^1$ = H or a protective group,

m = 1 or 2,

n = 1 or 2,

and salts thereof.

2. Sultines according to claim 1, satisfying formula 2, wherein $Y^1$ = H, t.butyloxycarbonyl or benzyloxycarbonyl, and acid addition salts thereof, wherein $Y^1$ = H.

3. Diastereomers of sultines according to claim 1 or 2.

4. Sultine $S_c$-$R_s$ diastereomer having formula 3, wherein $Y^1$ = H, t.butyloxycarbonyl or benzyloxycarbonyl, and acid addition salts thereof, in which $Y^1$ = H.

5. A process for preparing sultines according to one or more of claims 1-4, characterized by reacting a compound having formula 4, wherein

X = O or NH,

$Y^1$ = a protective group,

$Y^2$ = H or a group removable by oxidation,

$Y^3$ = H or a group removable by oxidation,

m = 1 or 2,

n = 1 or 2,

or the dimer having formula 5, wherein the symbols have the above meanings, in the presence of a weakly nucleophilic oxygen donor, with an oxidant comprising an oxidatively active part and a weakly nucleophilic

counter-ion, and if desired removing the protective group $Y^1$, or replacing it by a different protective group, and if desired separating the resulting mixture of diastereomers by column chromatography.

6. A process according to claim 5, characterized by using as the oxidant N-chloro- or N-bromo-succinimide, N-chlorobenztriazole, hypochlorous acid or tert.alkyl hypochlorite.

7. A process according to claim 6, characterized by using as the oxidant N-chloro- or N-bromo-succinimide.

8. A process according to any of claims 5-7, characterized by using as said oxygen donor a carboxylic acid, carboxylic acid anhydride, alcohol, or water.

9. A process according to claim 8, characterized by using as the oxygen donor acetic acid or acetic acid anhydride.

10. A process according to any of claims 5-9, characterized in that an excess of oxygen donor serves as a solvent.

11. A process according to any of claims 5-10, characterized by using an inert solvent.

12. A process according to claim 11, characterized by using as the solvent or as a solvent component an aromatic hydrocarbon, such as benzene or a halogenated hydrocarbon, such as $CH_2Cl_2$, $CHCl_3$, $CCl_4$, $CH_2Br_2$ etc.

13. A process according to any of claims 5-12, characterized by using a stereoisomer of a compound having formula 4 or 5.

14. A process according to any of claims 5-13, characterized by using a compound having formula 6, wherein $Y^1$ = t.butyloxycarbonyl or benzyloxycarbonyl, or the dimer thereof.

15. A process according to claim 14, characterized by using a compound having formula 6 or the dimer thereof, wherein $Y^1$ is t.butyloxy-

carbonyl, and the reaction is carried out in the presence of a non-nucleophilic base, such as pyridine or a trialkylamine.

16. A process according to claim 14,or 15, characterized by using the $S_c$ stereoisomer having formula 7 or the dimer thereof.

17. A process according to any of claims 5-16, characterized in that the resulting mixture of $S_s$ and $R_s$ sultine diastereomers is separated by column chromatography.

18. A process for preparing compounds having formula 8, wherein

X = O or NH,

$Y^1$ = H or a protective group,

$R^1$ = H, alkyl, acyl, or a protective group,

$R^2$ = a nucleophilic group,

m = 1 or 2,

n = 1 or 2,

characterized by reacting a sultine having formula 1, wherein $Y^1$ represents a protective group and the other symbols have the meanings specified in claim 1, at low temperatures and in an inert atmosphere with an organo-lithium compound $LiR^2$ in a solvent conventional for carb-anion reactions at low temperatures, and if desired providing the alcohol function with a protective group or etherifying or esterifying it, and if desired removing the protective group $Y^1$ or replacing it by a different protective group.

19. A process according to claim 18, characterized in that $R^2$ is selected from alkyl, alkoxyalkyl, alkoxyaryl, alkylthioalkyl, alkylthioaryl, alkylthio-oxoalkyl, alkylthio-oxoaryl, alkylthio-dioxoalkyl, and alkylthio-dioxoaryl.

20. A process according to claim 18 or 19, characterized by using

 

a mixture of- sultine diastereomers and separating the resulting mixture of $S_s$ and $R_s$ diastereomers of the compound having formula 8 by column chromatography.

21. A process according to claim 18 or 19, characterized by using a specific sultine diastereomer.

22. A process according to any of claims 18-21, characterized by preparing a compound having formula 9 by starting from a sultine having formula 2, wherein $Y^1$ = t.butyloxycarbonyl or benzyloxycarbonyl.

23. A process according to claim 22, characterized by preparing the $S_c$-$R_s$ diastereomer having formula 10.

24. A process for preparing sparsomycin and related compounds having formula 11, wherein $R^1$ = H, alkyl, acyl, and $R^2$ has the meanings specified in claim 19, which comprises reacting a compound having formula 12 or a suitable derivative thereof with a compound having formula 9, wherein $Y^1$ = H and $R^1$ and $R^2$ have the meanings specified, said compound being produced by the process according to claim 22 or 23.

25. A process according to claim 24, characterized by preparing one of the trans-diastereomers $S_c$-$R_s$, $S_c$-$S_s$, $R_c$-$R_s$, $R_c$-$S_s$ of the compound having formula 11 by starting from the corresponding diastereomer of the compound having formula 9, and if desired converting the resulting trans-diastereomer into the cis-diastereomer by irradiation.

26. A process according to claim 25, characterized by preparing trans-$S_c$-$R_s$-sparsomycin having formula 13.

1

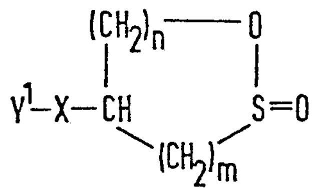

2

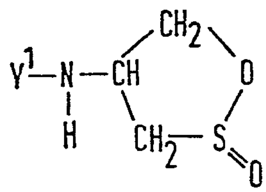

3

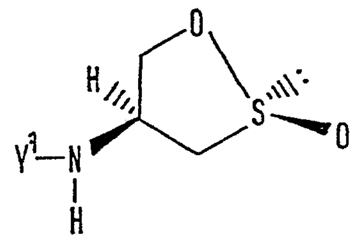

4

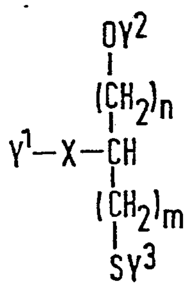

5

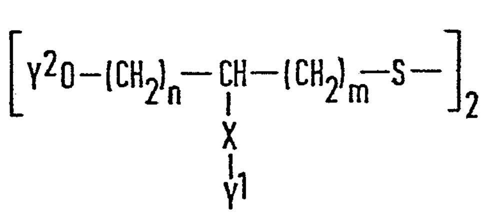

6

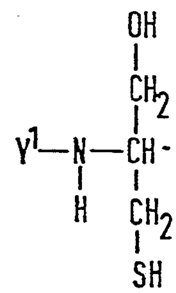

7

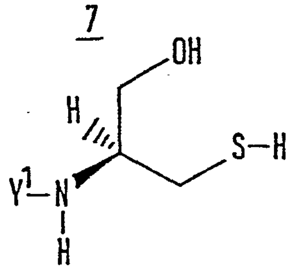

8

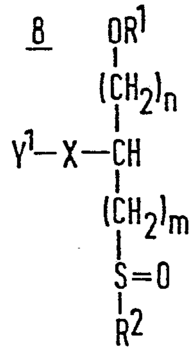

9

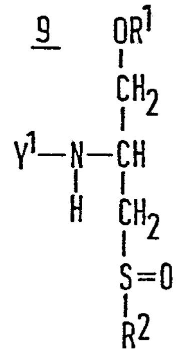

10

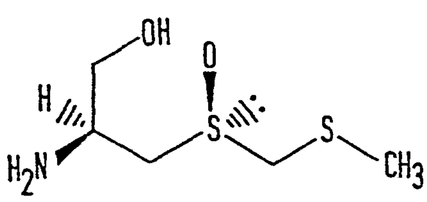

11

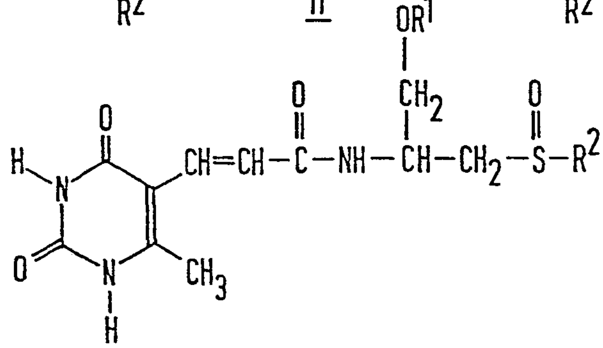

12

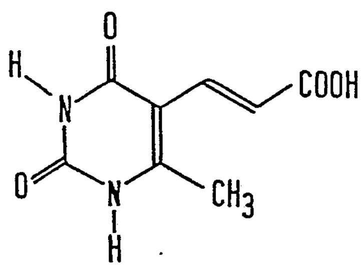

13

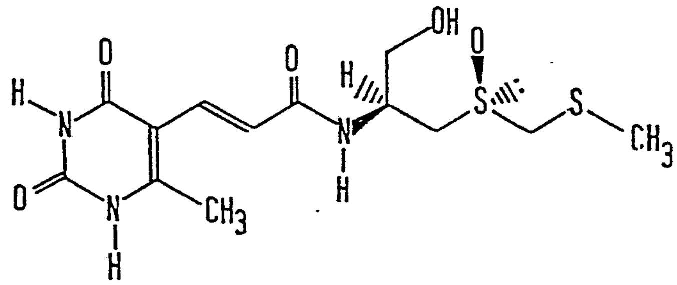

14

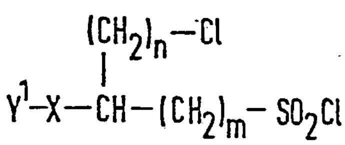

15

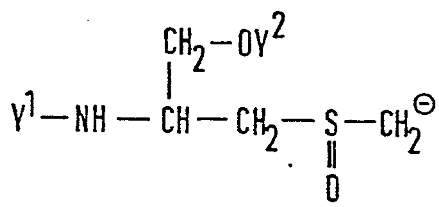

16

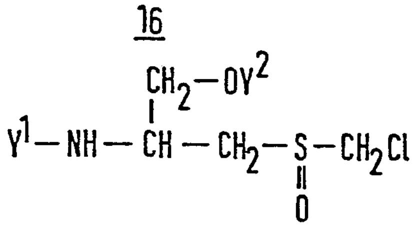

17

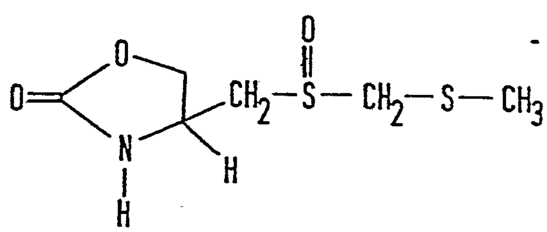

18

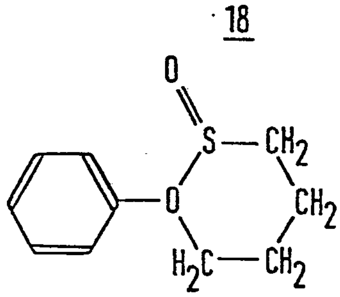

19